# EUROPEAN PATENT APPLICATION

(11) **EP 2 108 329 A2**
(43) Date of publication of application: **14.10.2009**
(21) Application number: 09005099.8
(22) Date of filing: 07.04.2009
(51) Int. Cl.: A61B 19/00

(54) **Medical manipulator system**

(30) Priority: 07.04.2008 JP 2008099550
(71) Applicant: Olympus Medical Systems Corp., Tokyo 151-0072 (JP)
(72) Inventor: Yoshie, Michifumi, Tokyo 151-0072 (JP); Banju, Kazuo, Tokyo 151-0072 (JP)
(74) Representative: von Hellfeld, Axel

(57) **Abstract**

A medical manipulator system of the present invention includes: a medical instrument having a shape insertable into an elongated tube passage and in which one or a plurality of flexion portions are provided; a flexing actuator for supplying a driving force to flex the flexion portion; an actuator control section for controlling the flexing actuator; a detecting section provided inside the tube passage to output a detection signal when detecting that the flexion portion passes therethrough; and a computing section for performing computation to match a flexed state of the flexion portion passing through the detecting section to an operating state of an operation portion operable to flex the flexion portion based on an operation amount of the flexing actuator detected when the detection signal is inputted.

## Description

### BACKGROUND OF THE INVENTION

### 1. Field of the Invention

The present invention relates to a medical manipulator system, and more particularly, to a medical manipulator system for use in treatment of various organs in a living body.

### 2. Description of the Related Art

Conventionally, endoscopes have been widely used in an industrial field, a medical field or the like. In particular, endoscopes in the medical field are mainly used when various organs in a living body are observed, treated or the like.

Meanwhile, at the time of treating various organs in a living body by use of the endoscope, a treatment instrument such as a forceps having an elongated shape, for example, in accordance with a shape of the endoscope is used in combination therewith.

Furthermore, as one of the treatment instruments for facilitating approach to a desired area in a living body as a treatment target, a medical manipulator disclosed in Japanese Patent Application Laid-Open Publication No. 08-224248, which includes bendable (capable of being flexed) bending portions (flexion portions) at a plurality of positions of a distal end portion of the treatment instrument and can change a bending (flexed) state of each of the bending portions (the flexion portions) in accordance with operation of a controller, has been proposed, for example.

The medical manipulator disclosed in Japanese Patent Application Laid-Open Publication No. 08-224248 includes a slave side manipulator having two bending portions in a longitudinal direction of each arm of a grasping forceps that is provided at the distal end portion, and a master side manipulator as the controller, and has a configuration in which the bending state of each of the bending portions of the slave side manipulator can be changed in accordance with operation of the master side manipulator.

The medical manipulator disclosed in Japanese Patent Application Laid-Open Publication No. 08-224248 is used by inserting the slave side manipulator along with an endoscope into a sheath, and projecting the grasping forceps of the slave side manipulator (and a distal end portion of the endoscope) from a distal end side of the sheath.

Thus, in the medical manipulator disclosed in Japanese Patent Application Laid-Open Publication No. 08-224248, it is necessary to perform an initializing process to match a bending state of the grasping forceps (each arm thereof) to a bending state of the grasping forceps (each arm thereof) recognizable by an operator based on an operating state of the master side manipulator in advance before treatment of a desired area. As a result, there occurs such a problem that it takes long time for the treatment of the desired area.

The present invention has been made in view of the aforementioned circumstances, and it is an object of the present invention to provide a medical manipulator system capable of reducing an amount of time required for treatment of a desired area to be shorter than ever before.

### SUMMARY OF THE INVENTION

A medical manipulator system according to the present invention includes: a medical instrument having a shape insertable into an elongated tube passage and in which one or a plurality of flexion portions are provided; a flexing actuator for supplying a driving force to flex the flexion portion; an actuator control section for controlling the flexing actuator; a detecting section provided inside the tube passage to output a detection signal when detecting that the flexion portion passes therethrough; and a computing section for detecting an operation amount of the flexing actuator when the detection signal is inputted, and performing computation to match a flexed state of the flexion portion passing through the detecting section to an operating state of an operation portion operable to flex the flexion portion based on the operation amount.

A medical manipulator system according to the present invention includes: a medical instrument having a shape insertable into an elongated tube passage and in which one or a plurality of flexion portions are provided; a flexing actuator for supplying a driving force to flex the flexion portion; an inserting actuator for supplying a driving force to insert the medical instrument into the tube passage; an actuator control section for controlling the flexing actuator and the inserting actuator; and a computing section for detecting an operation amount of the flexing actuator when the medical instrument is inserted a predetermined amount into the tube passage by the inserting actuator, and performing computation to match a flexed state of the flexion portion passing through the detecting section to an operating state of an operation portion operable to flex the flexion portion based on the operation amount.

A medical manipulator system according to the present invention includes: a medical instrument having a shape insertable into an elongated tube passage and in which one or a plurality of flexion portions are provided; a flexing actuator connected to the flexion portion by a wire to supply a driving force to flex the flexion portion via the wire; a detecting section provided inside the tube passage to output a detection signal when detecting that the flexion portion passes therethrough; an actuator control section for performing control to drive the flexing actuator until slack in the wire is removed when the detection signal is inputted; and a computing section for detecting a drive amount of the flexing actuator in accordance with control of the actuator control section, and performing computation to match a flexed state of the flexion portion passing through the detecting section to an operating state of an operation portion operable to flex the flexion portion based on the drive amount.

A medical manipulator system according to the present invention includes: a medical instrument having a shape insertable into an elongated tube passage and in which one or a plurality of flexion portions are provided; an inserting actuator for supplying a driving force to insert the medical instrument into the tube passage; a flexing actuator connected to the flexion portion by a wire to supply a driving force to flex the flexion portion via the wire; an actuator control section for controlling the inserting actuator and performing control to drive the flexing actuator until slack in the wire is removed when the medical instrument is inserted a predetermined amount into the tube passage by the inserting actuator; and a computing section for detecting an operation amount of the flexing actuator in accordance with control of the actuator control section when the medical instrument is inserted the predetermined amount into the tube passage by the inserting actuator, and performing computation to match a flexed state of the flexion portion passing through the detecting section to an operating state of an operation portion operable to flex the flexion portion based on the operation amount.

A medical manipulator system according to the present invention includes: a tubular medical instrument having a diameter insertable into a tubular body cavity, and a flexible portion capable of being flexed and having flexibility; a tube passage extending eccentrically relative to a central axis of the medical instrument; a wire inserted into the tube passage in a forward/backward movable manner; a moved portion moved in accordance with forward/backward movement of the wire to perform observation or treatment; a first detecting section for detecting a relative moving amount of the wire with respect to the tube passage, the moving amount being generated in accordance with flexion of the flexible portion; a second detecting section for detecting that the medical instrument is inserted into the tubular body cavity; and a control section capable of controlling the forward/backward movement of the wire in a direction in which the moving amount is reduced based on detection results of the first and second detecting sections.

### BRIEF DESCRIPTION OF THE DRAWINGS

Fig. 1 is a view showing an example of a configuration of main parts of a medical manipulator system according to an embodiment of the present invention;
Fig. 2 is a view showing an example of a configuration of a distal end surface of a distal end portion of an endoscope in Fig. 1;
Fig. 3 is a view showing a state in which a manipulator arm of a slave side manipulator is inserted into a treatment instrument channel provided in an endoscope;
Fig. 4 is a view showing an example of a flexed state of a flexion portion at the time of acquiring information related to a first motor rotation amount (rotation angle);
Fig. 5 is a view showing an example of a flexed state of a flexion portion at the time of acquiring information related to a second motor rotation amount (rotation angle);
Fig. 6 is a view showing a state in which a manipulator arm of a slave side manipulator is projected from a treatment instrument projection opening;
Fig. 7 is a view showing an example of a case in which sensors are provided around an inlet of a treatment instrument insertion opening;
Fig. 8 is a view showing an example of a configuration of main parts in a case where a treatment instrument insertion device is applied to the medical manipulator system in Fig. 1;
Fig. 9 is a view showing an example of a configuration of main parts of a medical manipulator system according to a first modification of the embodiment of the present invention;
Fig. 10 is a view showing an example of a configuration of main parts of a medical manipulator system according to a second modification of the embodiment of the present invention;
Fig. 11 is a view showing an example of a configuration of main parts of a medical manipulator system according to a third modification of the embodiment of the present invention;
Fig. 12 is a view showing an example of a configuration of main parts of a medical manipulator system according to a fourth modification of the embodiment of the present invention; and
Fig. 13 is a view showing an example of a configuration of main parts of a medical manipulator system according to a fifth modification of the embodiment of the present invention.

### DETAILED DESCRIPTION OF THE PREFERRED EMBODIMENT(S)

Embodiments of the present invention will be described below with reference to the drawings.

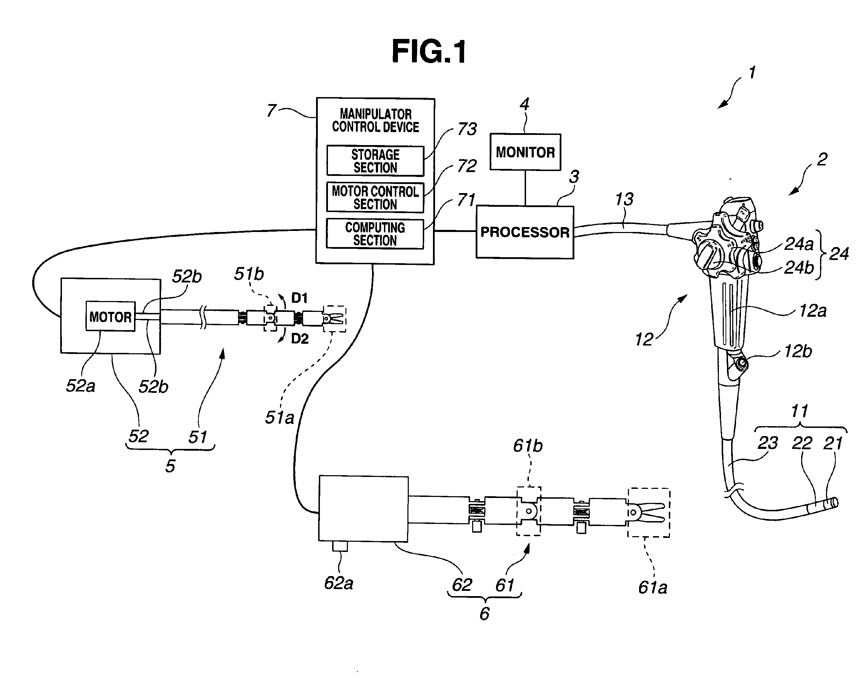

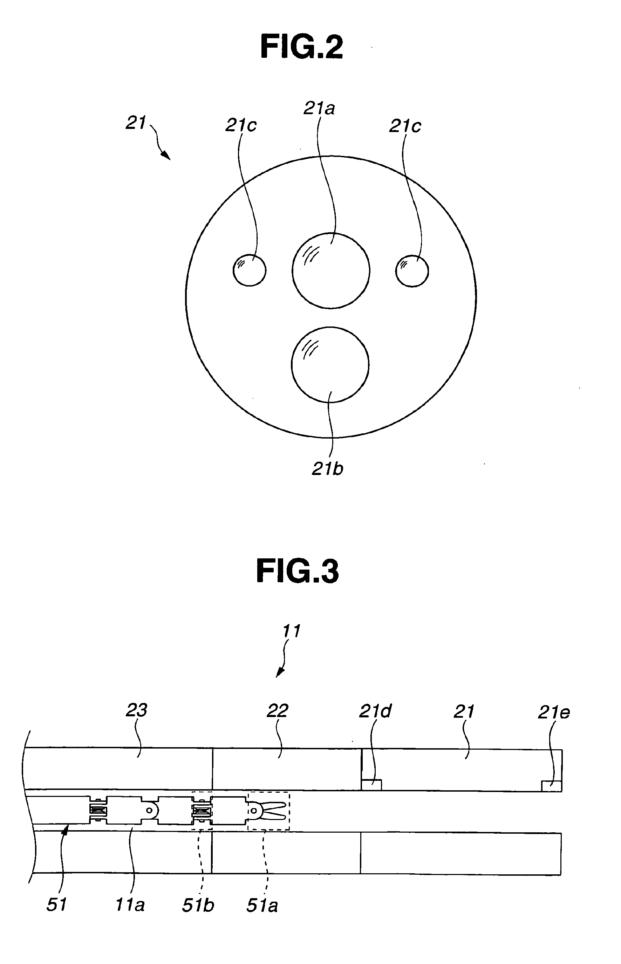

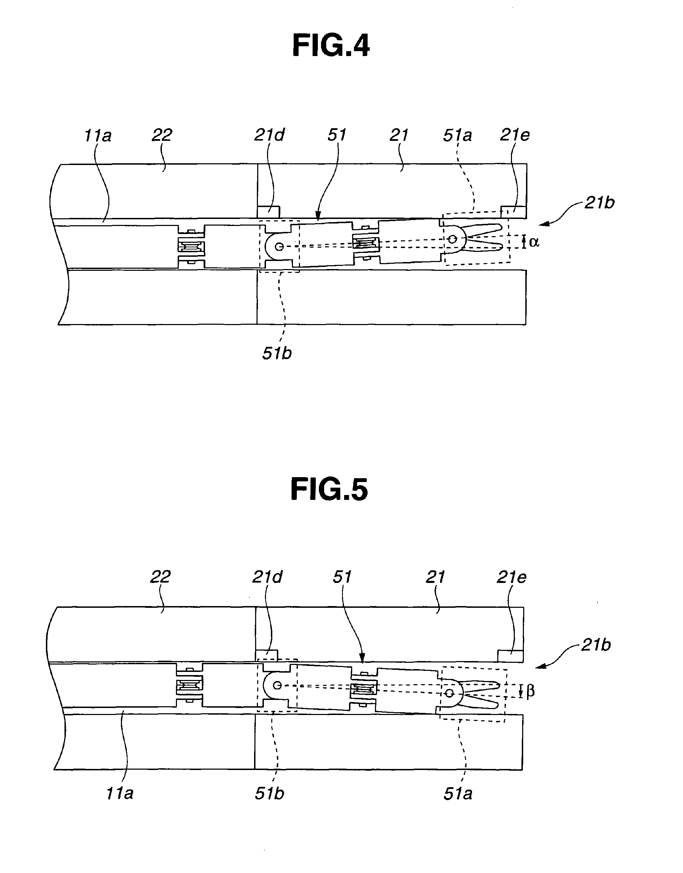

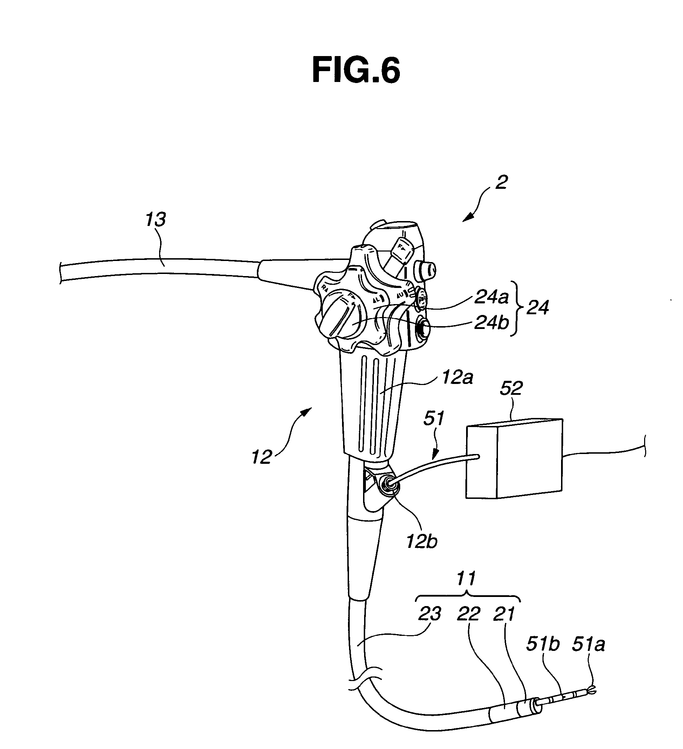

Figs. 1 to 13 are related to the embodiments of the present invention. Fig. 1 is a view showing an example of a configuration of main parts of a medical manipulator system according to an embodiment of the present invention. Fig. 2 is a view showing an example of a configuration of a distal end surface of a distal end portion of an endoscope in Fig. 1. Fig. 3 is a view showing a state in which a manipulator arm of a slave side manipulator is inserted into a treatment instrument channel provided in an endoscope. Fig. 4 is a view showing an example of a flexed state of a flexion portion at the time of acquiring information related to a first motor rotation amount (rotation angle). Fig. 5 is a view showing an example of a flexed state of a flexion portion at the time of acquiring information related to a second motor rotation amount (rotation angle). Fig. 6 is a view showing a state in which a manipulator arm of a slave side manipulator is projected from a treatment instrument projection opening.

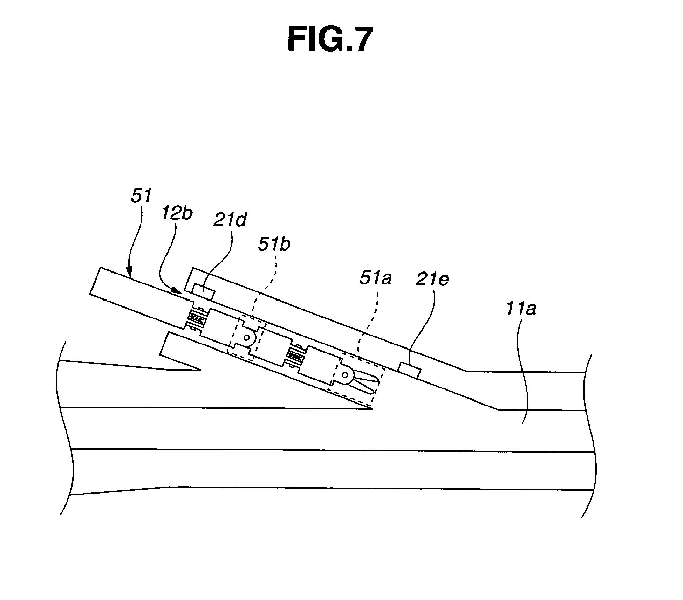

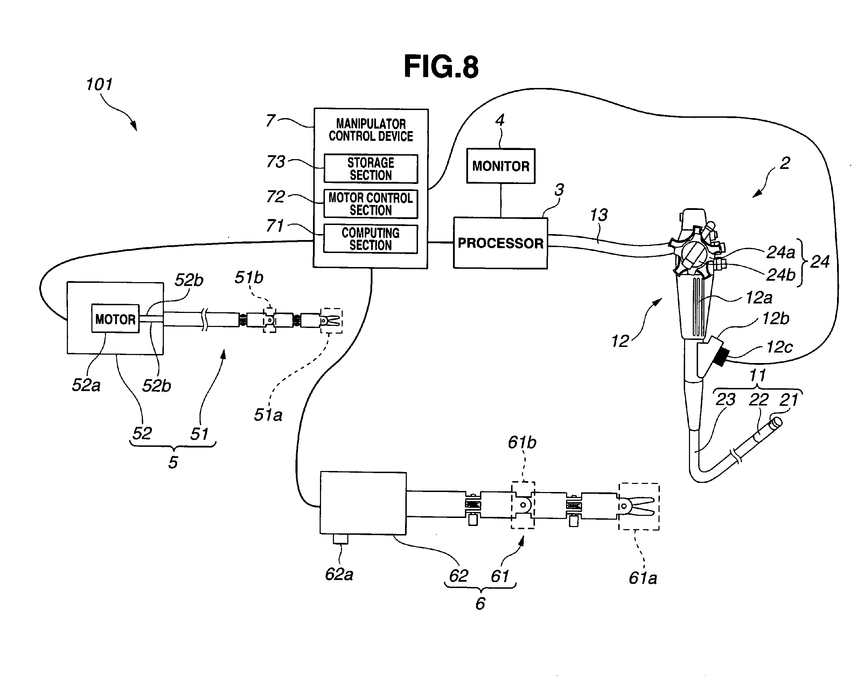

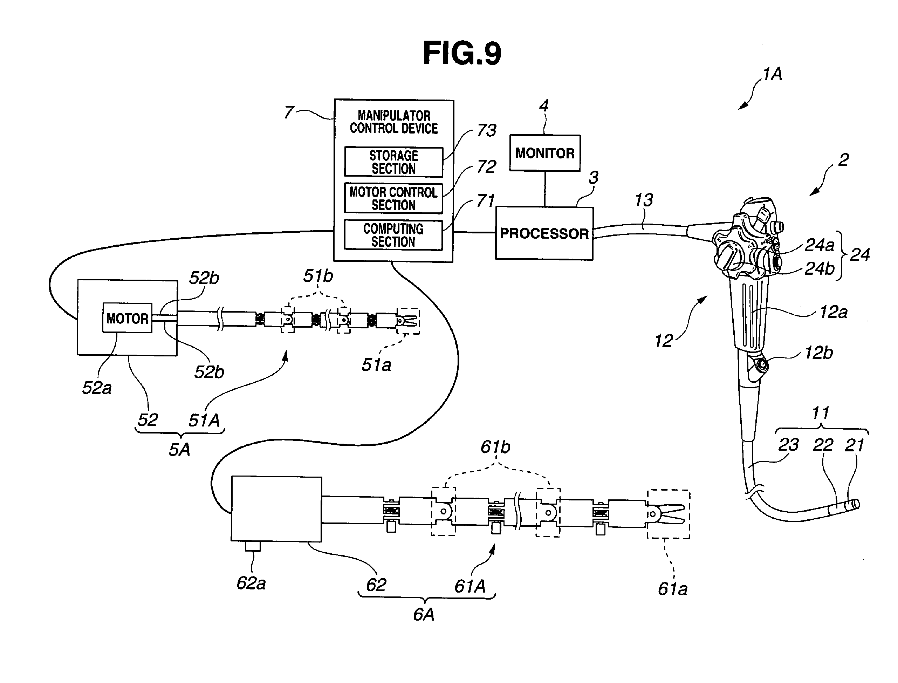

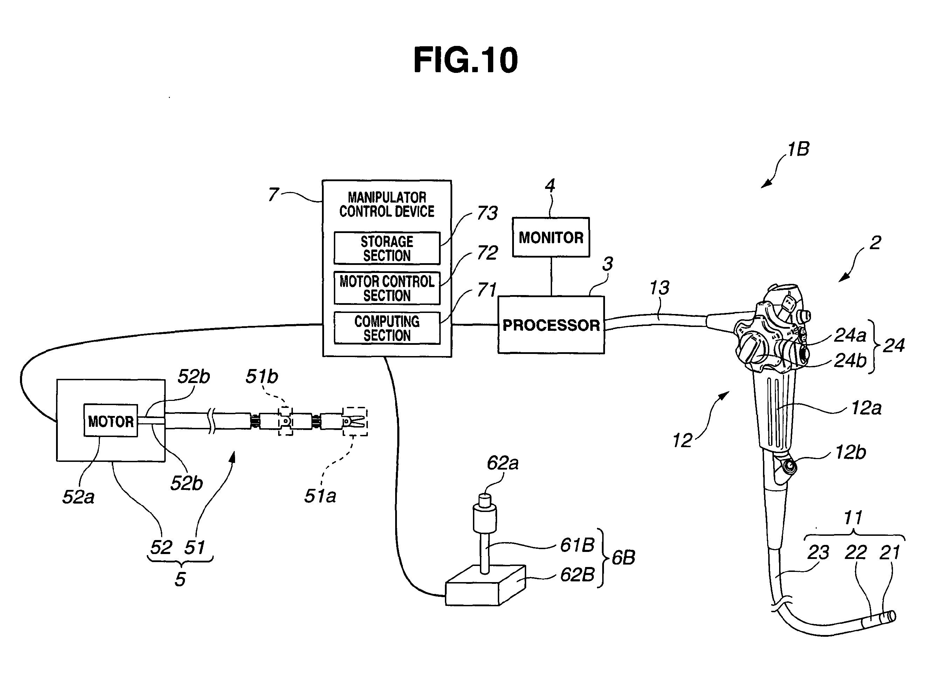

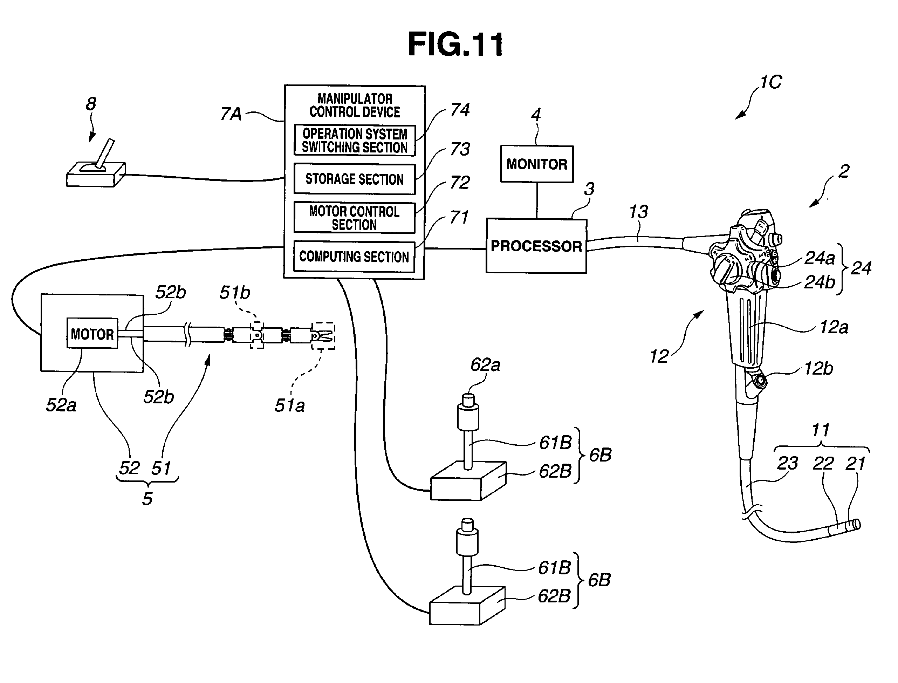

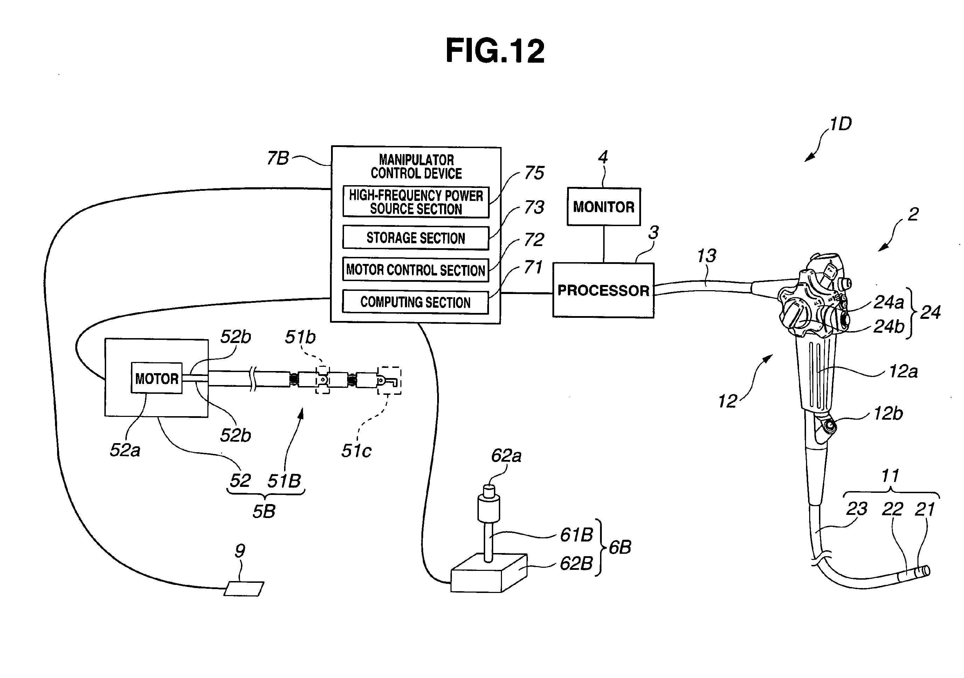

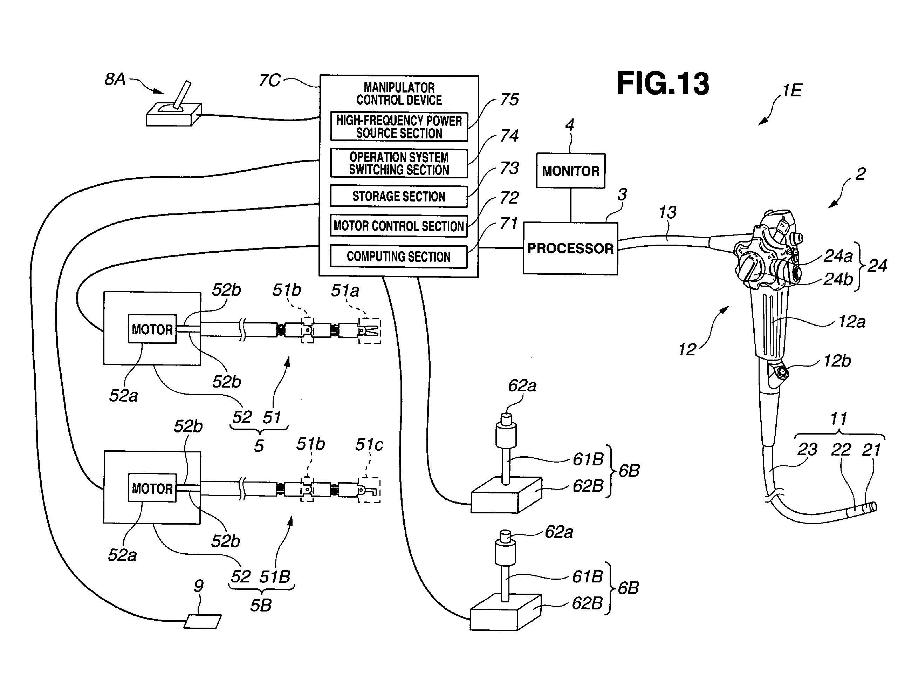

Fig. 7 is a view showing an example of a case in which sensors are provided around an inlet of a treatment instrument insertion opening. Fig. 8 is a view showing an example of a configuration of main parts in a case where a treatment instrument insertion device is applied to the medical manipulator system in Fig. 1. Fig. 9 is a view showing an example of a configuration of main parts of a medical manipulator system according to a first modification of the embodiment of the present invention. Fig. 10 is a view showing an example of a configuration of main parts of a medical manipulator system according to a second modification of the embodiment of the present invention. Fig. 11 is a view showing an example of a configuration of main parts of a medical manipulator system according to a third modification of the embodiment of the present invention. Fig. 12 is a view showing an example of a configuration of main parts of a medical manipulator system according to a fourth modification of the embodiment of the present invention. Fig. 13 is a view showing an example of a configuration of main parts of a medical manipulator system according to a fifth modification of the embodiment of the present invention.

A medical manipulator system 1 includes an endoscope 2 to be inserted into a living body, a processor 3, a monitor 4, a slave side manipulator 5, a master side manipulator 6, and a manipulator control device 7 as the main parts as shown in Fig. 1.

The endoscope 2 includes an insertion portion 11 having a shape and size insertable into a living body, an operation portion 12 having an endoscope grasping portion 12a that is connected to a proximal end side of the insertion portion 11, and a universal cable 13 whose one end extends from a side surface of the operation portion 12 and whose other end is removably connected to the processor 3.

In the insertion portion 11, a distal end portion 21 provided on a distal end side, a freely bendable bending portion 22 provided on the rear side of the distal end portion 21, and an elongated flexible tube portion 23 provided on the rear side of the bending portion 22 are provided in a coupled manner.

Although omitted in Fig. 1 and the like, an objective optical system 21a which forms an image of a subject, a treatment instrument projection opening 21b which communicates with a distal end side of a treatment instrument channel as a tube passage inserted into the insertion portion 11, and an illumination optical system 21c which emits illumination light that is supplied from an unillustrated light source are provided on a distal end surface of the distal end portion 21 as shown in Fig 2.

An unillustrated image pickup device which picks up the image of the subject formed by the objective optical system 21 a and outputs the image as an image pickup signal is incorporated in the distal end portion 21. The distal end portion 21 is formed of a relatively rigid member such as plastic, for example.

A treatment instrument channel 11a of the insertion portion 11 has a shape into which a manipulator arm 51 of the slave side manipulator 5 can be inserted as shown in Fig. 3, for example. As shown in Fig. 3, a sensor 21d and a sensor 21e are provided inside the distal end portion 21 as detecting sections capable of detecting that respective portions of the manipulator arm 51 pass therethrough while an initialize switch 62a described below is ON.

The operation portion 12 includes a bending operation portion 24 having a bending operation knob 24a for performing bending operation of the bending portion 22 and a fixing lever 24b for fixing the bending operation knob 24a at a desired rotational position. The operation portion 12 further includes a treatment instrument insertion opening 12b which communicates with a proximal end side of the treatment instrument channel inside the insertion portion 11.

The processor 3 performs signal processing on the image pickup signal outputted through the universal cable 13 to convert the image pickup signal to a video signal, and outputs the video signal. The processor 3 can also mediate communication between the endoscope 2 and the manipulator control device 7.

The slave side manipulator 5 as a medical instrument includes the manipulator arm 51 having a shape to be inserted from the treatment instrument insertion opening 12b to pass through the treatment instrument channel inside the insertion portion 11 and be projected from the treatment instrument projection opening 21b, and a manipulator driving section 52 provided on a proximal end side of the manipulator arm 51 to drive the manipulator arm 51 in accordance with control of the manipulator control device 7.

The manipulator arm 51 has an elongated shape insertable into the treatment instrument channel inside the insertion portion 11, and includes a grasping forceps 51a and a flexion portion 51b.

The manipulator driving section 52 includes a motor 52a which rotates to generate a driving force, and a pair of wires 52b which transmit the driving force generated by the motor 52a to the respective portions of the manipulator arm 51.

The grasping forceps 51a provided on a distal end side of the manipulator arm 51 can hold and grasp an object by converting the driving force transmitted by the pair of wires 52b to a grasping force.

The flexion portion 51 b provided in mid-course of the manipulator arm 51 includes an unillustrated bending piece capable of pivoting in directions of D1 (a first direction) and D2 (a second direction) in Fig. 1 in accordance with the driving force transmitted by the pair of wires 52b.

The motor 52a as a flexing actuator rotates in accordance with the control of the manipulator control device 7, to generate the driving force to drive the respective portions of the manipulator arm 51. The motor 52a also outputs information about its rotation amount (its rotation angle) to the manipulator control device 7 via an unillustrated encoder.

The master side manipulator 6 includes a movable portion 61 operable in conjunction with the respective portions of the manipulator arm 51, and a master side control section 62 provided on a proximal end side of the movable portion 61 to continually monitor an operating state of the movable portion 61 and output the operating state to the manipulator control device 7.

The movable portion 61 includes a forceps operation portion 61 a provided on a distal end side of the movable portion 61 and operable in conjunction with the grasping forceps 51a, and a flexion operation portion 61 b provided in mid-course of the movable portion 61 and operable in conjunction with the flexion portion 51b.

The master side control section 62 includes the initialize switch 62a for giving an instruction to execute an initializing process of the slave side manipulator 5 on an outer surface.

The manipulator control device 7 includes a computing section 71 which performs various computing processes, a motor control section 72, and a storage section 73 capable of storing various data, computation results or the like.

The motor control section 72 as an actuator control section controls the motor 52a based on the operating state of the movable portion 61 continually outputted from the master side control section 62, to allow the respective portions of the manipulator arm 51 to operate in conjunction with the operating state.

Here, an operation of the medical manipulator system 1 according to the present embodiment will be described.

First, after starting up the respective parts of the medical manipulator system 1, an operator inserts the insertion portion 11 such that the distal end portion 21 reaches a desired area to be treated while viewing an image displayed on the monitor 4.

When confirming that the distal end portion 21 has reached the desired area to be treated based on the image displayed on the monitor 4, the operator turns ON the initialize switch 62a, and then, sequentially inserts the grasping forceps 51a and the flexion portion 51b of the manipulator arm 51 into the treatment instrument channel 11a inside the insertion portion 11 as shown in Fig. 3, for example.

Meanwhile, when the initialize switch 62a is turned ON, the master side control section 62 performs control to deform the movable portion 61 into a linear shape along a central axis and allow the respective portions of the movable portion 61 to be temporarily immovable (non-operable) in order to disable operation of the movable portion 61 during the initializing process. Also, when the initialize switch 62a is turned ON, the sensor 21d starts detecting whether the respective portions of the manipulator arm 51 pass through a proximal end side of the distal end portion 21 in the treatment instrument channel 11a. Furthermore, when the initialize switch 62a is turned ON, the sensor 21e starts detecting whether the respective portions of the manipulator arm 51 pass through a distal end side of the distal end portion 21 in the treatment instrument channel 11a.

The sensor 21 d outputs a detection signal when detecting that the flexion portion 51 b of the manipulator arm 51 has passed through the proximal end side of the distal end portion 21 in the treatment instrument channel 11a.

The detection signal outputted from the sensor 21d is inputted to the manipulator control device 7 through an unillustrated signal line provided inside the endoscope 2, the universal cable 13 and the processor 3.

The motor control section 72 of the manipulator control device 7 performs control on the manipulator driving section 52 to acquire information required for the initializing process of the slave side manipulator 5 based on the detection signal from the sensor 21d.

To be more specific, the motor control section 72 controls the motor 52a to allow the unillustrated bending piece of the flexion portion 51 b to pivot in the D 1 direction (the first direction) in Fig. 1 until slack in the pair of wires 52b is removed. In this case, the flexion portion 51b is flexed by an angle α with respect to a central axis of the manipulator arm 51 in a longitudinal direction as shown in Fig. 4, for example. At the same time, information related to a first motor rotation amount (rotation angle), which is the rotation amount (the rotation angle) of the motor 52a corresponding to the angle α, is outputted via the unillustrated encoder, and stored in the storage section 73 of the manipulator control device 7.

When detecting that the information related to the first motor rotation amount (rotation angle) is stored in the storage section 73, the motor control section 72 controls the motor 52a to allow the unillustrated bending piece of the flexion portion 5 1 b to pivot in the D2 direction (the second direction) in Fig. 1 until slack in the pair of wires 52b is removed. In this case, the flexion portion 51 b is flexed by an angle β with respect to the central axis of the manipulator arm 51 in the longitudinal direction as shown in Fig. 5, for example. At the same time, information related to a second motor rotation amount (rotation angle), which is the rotation amount (the rotation angle) of the motor 52a corresponding to the angle β, is outputted via the unillustrated encoder, and stored in the storage section 73 of the manipulator control device 7.

The information related to the first motor rotation amount (rotation angle) and the information related to the second motor rotation amount (rotation angle) are values obtained by taking as zero the rotation amount (the rotation angle) of the motor 52a when the manipulator arm 51 withdrawn from the treatment instrument channel 11a is vertically oriented.

On the other hand, the computing section 71 of the manipulator control device 7 calculates a standard motor rotation amount (rotation angle) as the rotation amount of the motor 52a set in accordance with a bending state of the bending portion 22 by dividing a value obtained by adding the first motor rotation amount (rotation angle) to the second motor rotation amount (rotation angle) by two based on the information related to the first motor rotation amount (rotation angle) and the information related to the second motor rotation amount (rotation angle) stored in the storage section 73, and stores the standard motor rotation amount in the storage section 73. The standard motor rotation amount may be considered as a value of a relative moving amount of the pair of wires 52b with respect to an unillustrated tube passage into which the pair of wires 52b is inserted, for example.

The computing section 71 of the manipulator control device 7 also calculates a corrected motor rotation amount as the rotation amount of the motor 52a set in accordance with a slack state of the pair of wires 52b by obtaining an absolute value of a value obtained by subtracting the second motor rotation amount (rotation angle) from the first motor rotation amount (rotation angle) based on the information related to the first motor rotation amount (rotation angle) and the information related to the second motor rotation amount (rotation angle) stored in the storage section 73, and stores the corrected motor rotation amount in the storage section 73.

The motor control section 72 performs control to adjust the rotation amount of the motor 52a when the flexion portion 51b is projected from the treatment instrument projection opening 21b as the initializing process by using the standard motor rotation amount and the corrected motor rotation amount stored in the storage section 73.

To describe one example, the motor control section 72 rotates the motor 52a such that the flexion portion 51 b is in a linear state by changing settings of the rotation amount (the rotation angle) of the motor 52a for causing the flexion portion 51b to pivot based on the standard motor rotation amount before the flexion portion 51b is projected from the treatment instrument projection opening 21b. In other words, the motor control section 72 rotates the motor 52a in a direction in which the relative moving amount of the pair of wires 52b with respect to the unillustrated tube passage into which the pair of wires 52b is inserted is reduced, the moving amount being generated when the flexion portion 51b is inserted into the flexed treatment instrument channel 11a.

The initializing process is performed at a timing immediately after the detection signal from the sensor 21e for indicating that the flexion portion 51 b has passed through the distal end side of the distal end portion 21 in the treatment instrument channel 11a is inputted to the motor control section 72.

The flexion portion 5 1 b is projected from the treatment instrument projection opening 21b in a linear shape along the central axis of the manipulator arm 51 (in a state in which the flexion portion 51 b is not flexed in any direction) by the initializing process as shown in Fig. 6, for example. That is, due to the initializing process performed while the initialize switch 62a is ON, a flexed state of the manipulator arm 51 when the flexion portion 51b is projected from the treatment instrument projection opening 21b is matched to a flexed state of the movable portion 61.

The present embodiment is not limited to the configuration in which the sensor 21 d is provided on the proximal end side of the distal end portion 21 and the sensor 21e is provided on the distal end side of the distal end portion. For example, as shown in Fig. 7, the sensor 21 d may be provided around an inlet of the treatment instrument insertion opening 12b formed of a relatively rigid member that is substantially the same member as that of the distal end portion 21, and the sensor 2 1 e may be provided around a position where the treatment instrument insertion opening 12b joins the treatment instrument channel 11a.

Also, the medical manipulator system 1 is not limited to the configuration in which the motor 52a and the flexion portion 51b are connected via the pair of wires 52b. For example, the motor 52a formed as a servo motor may be incorporated in the flexion portion 51b.

In this case, the computing section 71 detects the rotation amount of the motor 52a immediately after the detection signal from the sensor 21d is inputted, and computes the rotation amount of the motor 52a required for forming the flexion portion 51 b into a linear shape based on the rotation amount. The motor control section 72 performs the initializing process on the motor 52a based on the rotation amount substantially immediately after the detection signal from the sensor 21e is inputted, so that the flexed state of the manipulator arm 51 when the flexion portion 51b is projected from the treatment instrument projection opening 21b is matched to the flexed state of the movable portion 61.

As described above, the medical manipulator system 1 of the present embodiment has a configuration and operation in which the initializing process to match the flexed states in the slave side manipulator 5 and the master side manipulator 6 can be performed while the manipulator arm 51 is being inserted into the treatment instrument channel 11a. Therefore, the medical manipulator system 1 of the present embodiment can reduce an amount of time required for treatment of a desired area to be shorter than ever before.

The medical manipulator system 1 of the present embodiment is not limited to the configuration in which an insertion state of the slave side manipulator 5 is detected by the sensors 21d and 21e. For example, as shown in Fig. 8, a medical manipulator system 101 capable of detecting the insertion state by using a treatment instrument insertion device 12c may be also employed.

The medical manipulator system 101 has substantially the same configuration as the medical manipulator system 1 except that the treatment instrument insertion device 12c is attached near the treatment instrument insertion opening 12b and the sensors 21d and 21 e (not shown in Fig. 8) are removed therefrom.

The treatment instrument insertion device 12c having a function as an inserting actuator operates based on control of the motor control section 72, and includes therein a motor, a driving roller or the like, which are not shown, capable of supplying a driving force to insert the slave side manipulator 5 inserted from the treatment instrument insertion opening 12b into the treatment instrument channel 11a. The treatment instrument insertion device 12c also detects an insertion amount of the slave side manipulator 5 into the treatment instrument channel 11a based on a rotation amount of the unillustrated motor, and continually outputs information of the insertion amount as a detection result to the manipulator control device 7.

Here, an operation of the medical manipulator system 101 will be described. For the simplicity of description, the following description will be made by appropriately omitting the same portions as those described above.

When the initialize switch 62a is turned ON, the computing section 71 starts monitoring the insertion amount based on the information of the insertion amount outputted from the treatment instrument insertion device 12c. When detecting that the insertion amount reaches a predetermined amount (for example, an amount by which the slave side manipulator 5 can reach the distal end portion 21), the computing section 71 outputs the detection result to the motor control section 72.

The motor control section 72 performs the control described above, so that the first motor rotation amount and the second motor rotation amount are stored in the storage section 73.

On the other hand, the computing section 71 calculates the standard motor rotation amount and the corrected motor rotation amount based on the first motor rotation amount and the second motor rotation amount, and stores the standard motor rotation amount and the corrected motor rotation amount in the storage section 73. A computation method for calculating the standard motor rotation amount and the corrected motor rotation amount is the same as the method described above.

The motor control section 72 performs control to adjust the rotation amount of the motor 52a when the flexion portion 51b is projected from the treatment instrument projection opening 21b as the initializing process by using the standard motor rotation amount and the corrected motor rotation amount stored in the storage section 73.

The initializing process in the medical manipulator system 101 is performed at a timing almost immediately after the computing section 71 detects that the insertion amount of the slave side manipulator 5 reaches a predetermined amount after the initialize switch 62a is turned ON.

Also, the medical manipulator system 101 is not limited to the configuration in which the motor 52a and the flexion portion 51b are connected via the pair of wires 52b. For example, the motor 52a formed as a servo motor may be incorporated in the flexion portion 51 b. In this case, the computing section 71 detects the rotation amount of the motor 52a substantially immediately after the insertion amount of the slave side manipulator 5 reaches a predetermined amount, and computes the rotation amount of the motor 52a required for forming the flexion portion 51 b into a linear shape based on the rotation amount. Thereafter, the motor control section 72 performs the initializing process on the motor 52a based on the rotation amount, so that the flexed state of the manipulator arm 51 when the flexion portion 51b is projected from the treatment instrument projection opening 21b is matched to the flexed state of the movable portion 61.

According to the configuration and operation of the medical manipulator system 101 described above, the insertion state (the insertion amount) of the slave side manipulator 5 can be acquired from the treatment instrument insertion device 12c (without using the sensors 21 d and 21 e). Therefore, the medical manipulator system 101 can produce the same effect as that of the medical manipulator system 1 described above.

Here, various modifications which can be applied to the present embodiment will be described. The following description will be made by appropriately omitting portions already described as the configuration or operation of the medical manipulator system 1.

The medical manipulator system 1 of the present embodiment is not limited to the configuration with the slave side manipulator 5 in which the flexion portion 51b is provided at only one position. For example, as shown in Fig. 9, a medical manipulator system 1A having a slave side manipulator 5A in which the flexion portions 51b are provided at a plurality of positions may be also employed.

To be more specific, the medical manipulator system 1A as a first modification of the present embodiment includes the endoscope 2, the processor 3, the monitor 4, the slave side manipulator 5A, a master side manipulator 6A, and the manipulator control device 7 as the main parts.

The slave side manipulator 5A includes a manipulator arm 51A and the manipulator driving section 52. The flexion portions 51b are provided at a plurality of positions in the manipulator arm 51 A (In Fig. 9, only two portions, a flexion portion provided on a distalmost end side of the manipulator arm 51 A and a flexion portion provided on a proximalmost end side of the manipulator arm 51A, out of the respective flexion portions 51b are shown.).

The master side manipulator 6A includes a movable portion 61A and the master side control section 62. The flexion operation portions 61 b are provided at a plurality of positions in mid-course of the movable portion 61 A (In Fig. 9, only two portions, a flexion operation portion provided on a distalmost end side of the movable portion 61A and a flexion operation portion provided on a proximalmost end side of the movable portion 61 A, out of the respective flexion operation portions 61b are shown.).

In the medical manipulator system 1A, the same initializing process as the initializing process performed when the number of flexion portions 51 b is one is sequentially performed on each of the flexion portions 51b.

To be more specific, each time one flexion portion 51 b passes through the distal end portion 21 in the treatment instrument channel 11a, information related to a first motor rotation amount (rotation angle) and information related to a second motor rotation amount (rotation angle) corresponding to the flexion portion 51 b are acquired. A standard motor rotation amount and a corrected motor rotation amount corresponding to the flexion portion 51b are calculated. The rotation amount of the motor 52a when the flexion portion 51b is projected from the treatment instrument projection opening 21b is adjusted. Accordingly, even the manipulator arm 51A in which the flexion portions 51b are provided at a plurality of positions can be projected from the treatment instrument projection opening 21b with the respective flexion portions 51b having a linear shape along the central axis of the manipulator arm 51 A (in a state in which the respective flexion portions 5 1 b are not flexed in any direction).

Operations and the like of other portions than the aforementioned portions in the medical manipulator system 1 A are substantially the same as those in the medical manipulator system 1.

The medical manipulator system 1 of the present embodiment is not limited to the configuration in which the slave side manipulator 5 can be operated by the master side manipulator 6. For example, as shown in Fig. 10, a medical manipulator system 1B in which the slave side manipulator 5 can be operated by a joystick 6B may be also employed.

To be more specific, the medical manipulator system 1B as a second modification of the present embodiment includes the endoscope 2, the processor 3, the monitor 4, the slave side manipulator 5, the joystick 6B, and the manipulator control device 7 as the main parts.

The joystick 6B includes a lever 61B on which the initialize switch 62a is provided, and a base portion 62B having a shape to support the lever 61 B and connected to the manipulator control device 7 via a signal line.

The motor control section 72 in the medical manipulator system 1B controls the motor 52a based on an operation instruction in accordance with an inclined state of the lever 61 B, to allow the respective portions of the manipulator arm 51 to operate in conjunction with the operation instruction.

The medical manipulator system 1 B performs a process for matching the flexed state of the manipulator arm 51 to the inclined state of the lever 61 B as the initializing process.

To be more specific, when the initialize switch 62a is turned ON, the lever 61 B is temporarily locked in a neutral position, and information related to a first motor rotation amount (rotation angle) and information related to a second motor rotation amount (rotation angle) in the neutral position are acquired. A standard motor rotation amount and a corrected motor rotation amount in the neutral position are calculated. The rotation amount of the motor 52a when the flexion portion 51b is projected from the treatment instrument projection opening 21b is adjusted. Accordingly, matching is effected such that a shape of the flexion portion 51 b corresponding to the neutral position of the lever 61 B is in a linear shape along the central axis of the manipulator arm 51.

Operations and the like of other portions than the aforementioned portions in the medical manipulator system 1 B are substantially the same as those in the medical manipulator system 1.

The medical manipulator system 1B may be also configured as a medical manipulator system 1C as shown in Fig. 11, for example, in which one joystick 6B for operating the slave side manipulator 5 can be selected from a plurality of joysticks 6B.

To be more specific, the medical manipulator system 1C as a third modification of the present embodiment includes the endoscope 2, the processor 3, the monitor 4, the slave side manipulator 5, a plurality of joysticks 6B, a manipulator control device 7A, and a selector switch 8 capable of selecting one joystick 6B for operating the slave side manipulator 5 out of the plurality of joysticks 6B as the main parts (In Fig. 11, for the simplicity of illustration, the medical manipulator system 1C has two joysticks 6B. Also, the selector switch 8 may have a shape or the like other than the one shown in Fig. 11.).

The manipulator control device 7A includes the computing section 71, the motor control section 72, the storage section 73, and an operation system switching section 74 which performs switching operation such that only an operation instruction from the joystick 6B selected by the selector switch 8 is inputted to the motor control section 72.

Operations and the like of the main parts in the medical manipulator system 1C are substantially the same as those in the medical manipulator system 1B.

The medical manipulator system 1B is not limited to the configuration with the slave side manipulator 5 in which the grasping forceps 51 a is provided on the distal end side. For example, as shown in Fig. 12, a medical manipulator system 1D having a slave side manipulator 5B in which an active electrode 51 c capable of outputting a high-frequency current is provided on a distal end side may be also employed.

To be more specific, the medical manipulator system 1D as a fourth modification of the present embodiment includes the endoscope 2, the processor 3, the monitor 4, the slave side manipulator 5B capable of outputting a high-frequency current to a desired area to be treated, the joystick 6B, a manipulator control device 7B which supplies a high-frequency current to the slave side manipulator 5B, and a return electrode 9 which receives the high-frequency current passing through the desired area to be treated as the main parts.

The slave side manipulator 5B includes a manipulator arm 51B having the flexion portion 51b and the active electrode 51c which outputs the high-frequency current supplied from the manipulator control device 7B to a desired area to be treated, and the manipulator driving section 52.

The manipulator control device 7B includes the computing section 71, the motor control section 72, the storage section 73, and a high-frequency power source section 75 which supplies the high-frequency current to the slave side manipulator 5B and to which the high-frequency current received by the return electrode 9 is inputted.

The motor control section 72 in the medical manipulator system 1D controls the motor 52a based on the operation instruction in accordance with the inclined state of the lever 61 B, to allow the respective portions of the manipulator arm 51 B to operate in conjunction with the operation instruction.

Operations and the like of the main parts in the medical manipulator system 1D are substantially the same as those in the medical manipulator system 1B.

The medical manipulator system 1C may be also configured as a medical manipulator system 1E as shown in Fig. 13, for example, in which two slave side manipulators 5 and 5B can be used at the same time.

To be more specific, the medical manipulator system 1E as a fifth modification of the present embodiment includes the endoscope 2, the processor 3, the monitor 4, the slave side manipulators 5 and 5B, the plurality of joysticks 6B, a manipulator control device 7C, a selector switch 8A capable of respectively selecting the joysticks 6B for operating the slave side manipulators 5 and 5B out of the plurality of joysticks 6B, and the return electrode 9 as the main parts (The selector switch 8A may have a shape or the like other than the one shown in Fig. 13.).

The manipulator control device 7C includes the computing section 71, the motor control section 72, the storage section 73, the operation system switching section 74, and the high frequency power source section 75.

Operations and the like of the main parts in the medical manipulator system 1E are substantially the same as those in the medical manipulator system 1C.

In the present embodiment, the same effect as that of the medical manipulator system 1 described above can be obtained even when the aforementioned respective modifications are applied to the present embodiment (separately or in appropriate combination). Also, the aforementioned respective modifications may be appropriately applied to the configuration of the medical manipulator system 101.

In the respective medical manipulator systems described as the present embodiment and the modifications, a configuration in which the slave side manipulator is remotely operated via a network line or wireless communication, for example, may be also added.

Also, the initializing process in the present embodiment is not limited to be performed while the slave side manipulator is being inserted into the treatment instrument channel of the endoscope. For example, the initializing process may be also performed while the slave side manipulator 5 is being inserted into a trocar as a tube passage having the sensors 21d and 21 e, for example.

It goes without saying that the present invention is not limited to the aforementioned respective embodiments, but various modifications and applications may be made therein without departing from the spirit of the invention.

## Claims

1. A medical manipulator system comprising:
a medical instrument having a shape insertable into an elongated tube passage and in which one or a plurality of flexion portions are provided;
a flexing actuator for supplying a driving force to flex the flexion portion;
an actuator control section for controlling the flexing actuator;
a detecting section provided inside the tube passage to output a detection signal when detecting that the flexion portion passes therethrough; and
a computing section for detecting an operation amount of the flexing actuator when the detection signal is inputted, and performing computation to match a flexed state of the flexion portion passing through the detecting section to an operating state of an operation portion operable to flex the flexion portion based on the operation amount.

2. A medical manipulator system comprising:
a medical instrument having a shape insertable into an elongated tube passage and in which one or a plurality of flexion portions are provided;
a flexing actuator for supplying a driving force to flex the flexion portion;
an inserting actuator for supplying a driving force to insert the medical instrument into the tube passage;
an actuator control section for controlling the flexing actuator and the inserting actuator; and
a computing section for detecting an operation amount of the flexing actuator when the medical instrument is inserted a predetermined amount into the tube passage by the inserting actuator, and performing computation to match a flexed state of the flexion portion passing through the detecting section to an operating state of an operation portion operable to flex the flexion portion based on the operation amount.

3. A medical manipulator system comprising:
a medical instrument having a shape insertable into an elongated tube passage and in which one or a plurality of flexion portions are provided;
a flexing actuator connected to the flexion portion by a wire to supply a driving force to flex the flexion portion via the wire;
a detecting section provided inside the tube passage to output a detection signal when detecting that the flexion portion passes therethrough;
an actuator control section for performing control to drive the flexing actuator until slack in the wire is removed when the detection signal is inputted; and
a computing section for detecting a drive amount of the flexing actuator in accordance with control of the actuator control section, and performing computation to match a flexed state of the flexion portion passing through the detecting section to an operating state of an operation portion operable to flex the flexion portion based on the drive amount.

4. A medical manipulator system comprising:
a medical instrument having a shape insertable into an elongated tube passage and in which one or a plurality of flexion portions are provided;
an inserting actuator for supplying a driving force to insert the medical instrument into the tube passage;
a flexing actuator connected to the flexion portion by a wire to supply a driving force to flex the flexion portion via the wire;
an actuator control section for controlling the inserting actuator and performing control to drive the flexing actuator until slack in the wire is removed when the medical instrument is inserted a predetermined amount into the tube passage by the inserting actuator; and
a computing section for detecting an operation amount of the flexing actuator in accordance with control of the actuator control section when the medical instrument is inserted the predetermined amount into the tube passage by the inserting actuator, and performing computation to match a flexed state of the flexion portion passing through the detecting section to an operating state of an operation portion operable to flex the flexion portion based on the operation amount.

5. The medical manipulator system according to any one of claims 1 to 4, wherein the actuator is a motor.

6. A medical manipulator system comprising:
a tubular medical instrument having a diameter insertable into a tubular body cavity, and a flexible portion capable of being flexed and having flexibility;
a tube passage extending eccentrically relative to a central axis of the medical instrument;
a wire inserted into the tube passage in a forward/backward movable manner;
a moved portion moved in accordance with forward/backward movement of the wire to perform observation or treatment;
a first detecting section for detecting a relative moving amount of the wire with respect to the tube passage, the moving amount being generated in accordance with flexion of the flexible portion;
a second detecting section for detecting that the medical instrument is inserted into the tubular body cavity; and
a control section capable of controlling the forward/backward movement of the wire in a direction in which the moving amount is reduced based on detection results of the first and second detecting sections.
